(19) Europäisches Patentamt
European Patent Office
Office européen des brevets

(11) Numéro de publication : **0 401 104 B1**

# (12) FASCICULE DE BREVET EUROPEEN

(45) Date de publication du fascicule du brevet : **11.08.93 Bulletin 93/32**

(51) Int. Cl.$^5$ : **C07C 69/63**, C07C 67/307

(21) Numéro de dépôt : **90401421.4**

(22) Date de dépôt : **29.05.90**

## (54) Procédé pour la préparation d'isomères optiques d'esters d'acide-2 chloropropionique.

(30) Priorité : **30.05.89 FR 8907370**

(43) Date de publication de la demande : **05.12.90 Bulletin 90/49**

(45) Mention de la délivrance du brevet : **11.08.93 Bulletin 93/32**

(84) Etats contractants désignés : **AT BE CH DE DK ES FR GB GR IT LI LU NL SE**

(56) Documents cités : **EP-A- 0 056 981**

(73) Titulaire : **RHONE-POULENC CHIMIE**
**25, quai Paul Doumer**
**F-92408 Courbevoie Cédex (FR)**

(72) Inventeur : **Metivier, Pascal**
**8, quai Jean Moulin**
**F-69001 Lyon (FR)**
Inventeur : **Rajoharisson, Harvelo**
**16, allée François Villon**
**F-38130 Echirolles (FR)**

(74) Mandataire : **Dutruc-Rosset, Marie-Claude et al**
**RHONE-POULENC CHIMIE Direction des Brevets 25, Quai Paul Doumer**
**F-92408 Courbevoie Cédex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 401 104 B1

## Description

L'invention se rapporte à un procédé de préparation d'esters d'acide 2-chloropropionique optiquement actifs à partir d'esters lactiques optiquement actifs correspondants avec inversion de configuration, en présence de chlorure de thionyle. Ces composés sont des intermédiaires chimiques bien connus permettant d'obtenir des produits chimiques utiles notamment comme herbicides. L'intérêt de préparer des herbicides optiquement actifs réside dans le fait que ceux-ci sont actifs à des doses moitié moindre que les composés racémiques correspondants. Il en découle un intérêt majeur en ce qui concerne notamment la sauvegarde de l'environnement.

De nombreux procédés ont déjà été proposés afin de réaliser cette synthèse avec les rendements les plus élevés possibles en utilisant le chlorure de thionyle. Ainsi le brevet français FR-B-2459221 décrit la chloration de l'ester lactique d'alcoyle racémique ou optiquement actif en présence de chlorure de thionyle et d'une base organique en maintenant dans le mélange réactionnel un excès molaire de chlorure de thionyle d'au moins 2,5 % par rapport à la quantité de lactate d'alcoyle introduit dans le mélange, à une température maintenue inférieure à la température de décomposition du chlorosulfite de lactate d'alcoyle pendant cette première étape puis en chauffant dans une deuxième étape le mélange réactionnel résultant de la première étape à une température au moins égale à la température de décomposition du chlorosulfite de lactate d'alcoyle.

Le brevet britannique GB 2055802 décrit l'obtention d'esters d'acides α-Chloro ou α-bromo propionique optiquement actifs qui comprend la réaction d'ester acide (L)- lactique avec du chlorure ou du bromure de thionyle

a) en présence d'une base pour donner l'ester de l'acide (D) α-chloro bromo propionique avec inversion de configuration ou

b) en l'absence d'une base pour donner un ester d'acide (L) α-chloro ou bromo propionique avec rétention de configuration.

Par ailleurs, on connait par la référence : Cram et Hammond, "Organic Chemistry", Mr graw HILL, 1959, pages 236-239, la réaction du 2-octanol optiquement actif avec $SOCl_2$ pour donner le 2-octyle clorosulfite qui est ensuite décomposé dans le dioxanne pour conduire au chloro-2 octane avec 68 % de rétention de configuration et 32 % de racémisation.

Il a maintenant été trouvé de manière inattendue qu'il était possible de préparer l'ester de 2-chloropropionate optiquement actif de formule :

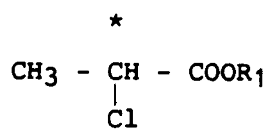

I

$COOR_1$ étant un groupe hydrolysable à partir d'un lactate optiquement actif de formule

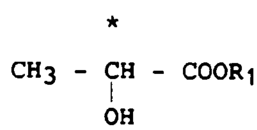

II

avec inversion de configuration par mise en contact du lactate (II) avec $SOCl_2$ puis décomposition du chlorosulfite obtenu, au moins l'étape de décomposition étant effectuée en présence d'un éther.

Le groupement $R_1$ est notamment choisi parmi les radicaux suivants :

- $C_1$-$C_{18}$ alkyle linéaire ou ramifié, de préférence $C_1$-$C_{12}$
- $C_2$-$C_{18}$ alcényle linéaire ou ramifié, de préférence $C_2$-$C_{12}$
- $C_2$-$C_{18}$ alcynyle linéaire ou ramifié, de préférence $C_2$-$C_{12}$
- $C_3$-$C_{18}$ cycloalkyle linéaire ou ramifié, de préférence $C_3$-$C_{12}$
- $C_6$-$C_{14}$ aryle, de préférence $C_6$-$C_{10}$
- $C_7$-$C_{15}$ aralkyle linéaire ou ramifié, de préférence $C_7$-$C_{11}$

ces radicaux étant éventuellement substitués par un ou plusieurs atomes d'halogène, radicaux $C_1$-$C_6$ alcoxy ou alkylthio, les cycles aromatiques des radicaux aryle ou aralkyle pouvant, en outre, comporter en remplacement d'un à quatre atomes de carbone, respectivement un à quatre hétéroatomes choisis parmi l'atome d'oxygène, de soufre, d'azote, (par exemple furyle, thiophényle, piridyle).

De préférence $R_1$ est un radical $C_1$-$C_6$ alkyle.

Parmi les ethers ou peut citer les ethers aliphatiques à chaine ouverte comme les ethers suivants :
Ethylvinyl ether,
diethyl ether, sulfuric ether, 3-oxapentane
di-n-propyl ether,
diisopropyl ether,
Butylvinyl ether,
Butylethyl ether,
di-n-amyl ether, 1-pentoxypentane,
Diisopentyl ether, diisoamyl ether 1,2-Dimethoxyethane,
Ethylene glycol dimethyl ether,
Diethylene glycol dimethyl ether.

Les ethers aliphatiques à chaines fermées comme les ethers suivants :
Ethyl oxyde, propylene oxide, 2-methyloxacyclopropane
1,2-Epoxybutane
1,8-epoxy-p-menthane, 1,3,3-trimethyl-2-oxabicyclo
(2.2.2) octane
Furan, furfuran, divinylene oxide,
Tetrahydrofuran, oxacyclopentane,
p-Dioxane, 1,4-dioxane, 1,3-dioxane,
Tetrahydropyran,
les ethers aromatiques comme les ethers suivants :
Benzylethyl ether, α-ethoxytoluene
Methoxybenzene,
ethoxybenzene,
dibenzyl ether, diphenyl ether,
O-dimethoxybenzene.

On peut opérer de deux manières :

En premier lieu il est possible de faire réagir directement le lactate (II) sur le chlorure de thionyle afin d'obtenir le chlorosulfite

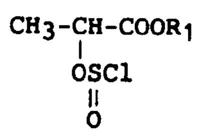

III

puis dans une seconde étape, de décomposer à une température appropriée le chlorosulfite III en présence d'un éther.

Le rapport molaire $SOCl_2$ : II (lactate) est généralement compris entre 0,8 et 2 et de préférence supérieur à 1,05.

La température peut varier de 0°C à l'ébullition de $SOCl_2$ de préférence supérieure à 20°C.

En ce qui concerne la seconde étape, généralement, sans que cela constitue une caractéristique critique la dilution en poids du chlorosulfite par rapport à l'éther est comprise entre 5 et 95 % en volume.

La température de décomposition est généralement comprise entre 80°C et la température d'ébullition du solvant ou inférieure à 140°C si le solvant a une température d'ébullition supérieure.

En second lieu et c'est un mode préféré, on met en contact le lactate (II), le chlorure de thionyle et l'éther, dans les mêmes conditions et avec des proportions identiques au mode d'exécution précédent, le chlorosulfite étant remplacé par le lactate pour la dilution avec l'éther pour les indications de proportions, à savoir :
$SOCl_2$ : II en mole généralement compris entre 0,8 et 2, et de préférence supérieur à 1,05.
Lactate : éther, dilution en poids compris entre 5 % et 95 %.

Après mélange des trois constituants, on chauffe à une température identique au mode d'exécution précédent

Exemple 1

Réactifs.

| | | | |
|---|---|---|---|
| D-lactate de methyle (96,7 % en enantiomètre D) | | | |
| | 5 ml | 5,45 g | 0,052 mole |
| $SOCl_2$ | | 9,3 g | 0,078 mole |
| diglyme seché sur tamis | 15 ml | 14,09 g | |

Mode opératoire :

On charge le diglyme et $SOCl_2$ dans un tricol de 50 ml sous argon, muni d'une agitation magnétique, d'un condenseur et d'un thermomètre. On coule le lactate en 35 mn à température ambiante et l'on chauffe à 100°C pendant 10 h.

Le dosage CPV du brut réactionnel montre un taux de transformation de 62,3 % et un rendement réel dosé en chloropropionate de 43,2 %. Le brut réactionnel est repris par 30 ml de $CH_2Cl_2$ puis lavé avec 3 x 15 ml d'eau, seché sur $Na_2SO_4$ filtré et évaporé sous vide. L'analyse CPV chirale de ce mélange réactionnel montre que le chloropropionate obtenu contient 95,4 % de l'énantiomètre L et 4,6 % de l'énantiomètre D soit un rendement énantiomèrique de 98 %.

Exemple 2

Réactifs :

| | | | |
|---|---|---|---|
| D-lactate de methyle (96,7 % en enantiomètre D) | | | |
| | 5 ml | 5,45 g | 0,052 mole |
| $SOCl_2$ | | 7,4 g | 0,062 mole |
| Dioxanne (sur tamis) | 15 ml | 15,9 g | |

Mode opératoire

Dans un tricol de 50 ml, muni d'un condenseur, d'un thermomètre, d'une agitation magnétique on charge le dioxanne. On coule doucement le $SOCl_2$ (on observe une légère exothermie) une fois la température revenue à 25°C on coule le lactate en 1 h. On chauffe ensuite le mélange réactionnel à 100°C pendant 26h.

Le dosage du brut réactionnel par chromatographie en phase vapeur montre: un taux de transformation de 60,6 % et un rendement réel dosé de 15,7 %. Une chromatographie bidimensionnelle effectuée sur le brut réactionnel montre que le chloropropionate obtenu a une teneur en isomere L de 82,4 %. soit un rendement énantiomérique de 65 %.

## Revendications

1. Procédé de préparation d'esters d'acide 2-chloropropionique optiquement actifs de formule

```
        *
CH3-CH-COOR1        I
    |
    Cl
```

$COOR_1$ étant un groupe hydrolysable à partir d'un lactate optiquement actif de formule

$$\overset{*}{\phantom{CH}}$$
$$CH_3\text{-}\underset{OH}{CH}\text{-}COOR^1 \qquad II$$

avec inversion de configuration par mise en contact du lactate (II) avec $SOCl_2$ puis décomposition du chlorosulfite formé, ledit procédé étant caractérisé en ce que au moins l'étape de décomposition est effectuée en présence d'un éther.

**2.** Procédé selon la revendication 1, caractérisé en ce que
Le groupement $R_1$ est notamment choisi parmi les radicaux suivants :
  $C_1$-$C_{18}$ alkyle linéaire ou ramifié, de préférence $C_1$-$C_{12}$
  $C_2$-$C_{18}$ alcényle linéaire ou ramifié, de préférence $C_2$-$C_{12}$
  $C_2$-$C_{18}$ alcynyle linéaire ou ramifié, de préférence $C_2$-$C_{12}$
  $C_3$-$C_{18}$ cycloalkyle linéaire ou ramifié, de préférence $C_3$-$C_{12}$
  $C_6$-$C_{14}$ aryle, de préférence $C_6$-$C_{10}$
  $C_7$-$C_{15}$ aralkyle linéaire ou ramifié, de préférence $C_7$-$C_{11}$
Ces radicaux étant éventuellement substitués par un ou plusieurs atomes d'halogène, radicaux $C_1$-$C_6$ alcoxy ou alkylthio, les cycles aromatiques des radicaux aryle ou aralkyle pouvant, en outre, comporter en remplacement d'un à quatre atomes de carbones, respectivement un à quatre hétéroatomes choisis parmi l'atome d'oxygène, de soufre, d'azote, (par exemple furyle, thiophényle, piridyle).

**3.** Procédé selon la revendication 2, caractérisé en ce que $R_1$ est un radical $C_1$-$C_6$ alkyle.

**4.** Procédé selon la revendication 1, caractérisé en ce que l'éther est choisi parmi le diglyme ou le dioxanne.

**5.** Procédé selon la revendication 1, caractérisé en ce que le rapport molaire lactate (II) par rapport au $SOCl_2$ est compris entre 0,8 et 2, et de préférence supérieure à 1,05.

**6.** procédé selon la revendication 1, caractérisé en ce que la température de décomposition est comprise entre 80°C et la température d'ébullition du solvant ou inférieure à 140°C si le solvant a une température d'ébullition supérieure.

**7.** Procédé selon l'une des revendications 1 à 6, caractérisé en ce que l'on met en contact dans une première étape le lactate (II) et le chlorure de thionyle afin d'obtenir le chlorosulfite de formule :

$$CH_3\text{-}\underset{OSOCl}{CH}\text{-}COOR_1 \qquad III$$

puis on décompose le chlorosulfite (III) en présence d'un éther.

**8.** Procédé selon la revendication 7, caractérisé en ce que la dilution en poids du chlorosulfite par rapport à l'éther est comprise entre 5 et 95%.

**9.** Procédé selon la revendication 1, caractérisé en ce qu'on met en contact le lactate (II), le chlorure de thionyle et l'éther puis en ce que l'on chauffe.

**10.** Procédé selon la revendication 9, caractérisé en ce que la dilution en poids du lactate par rapport à l'éther est comprise entre 5 et 95 %.

## Claims

**1.** A process for the preparation of optically active esters of 2-chloropropionic acid of the formula:

$$CH_3 - \overset{*}{C}HCl - COOR_1, \qquad (I)$$

where $COOR_1$ is a hydrolysable group, starting from an optically active lactate of formula:

$$CH_3 - \overset{*}{C}H(OH) - COOR_1 \qquad (II)$$

with inversion of configuration, by contacting the lactate (II) with thionyl chloride and decomposing the resulting chlorosulphite, characterised in that at least the decomposition step is effected in the presence of an ether.

2. A process according to Claim 1, characterized in that the group $R_1$ is a $C_{1-18}$ and particularly $C_{1-12}$ straight or branched alkyl, a $C_{2-18}$ and particularly $C_{2-12}$ straight or branched alkenyl, a $C_{2-18}$ and preferably $C_{2-12}$ straight or branched alkynyl, a $C_{3-18}$ and particularly $C_{3-12}$ straight or branched cycloalkyl, a $C_{6-14}$ and particularly $C_{6-10}$ aryl, or a $C_{7-15}$ and particularly $C_{7-11}$ straight or branched aralkyl radical, these radicals being optionally substituted by one or more halogen atoms and/or $C_{1-6}$ alkoxy and/or alkylthio radicals, and in which the aromatic rings of the aryl or aralkyl radicals can also possibly contain a replacement for one to four carbon atoms by one to four hetero atoms selected from oxygen, sulphur and nitrogen, (for example furyl,thiophenyl, pyridyl).

3. A process according to Claim 2, characterized in that $R_1$ is a $C_{1-6}$ alkyl radical.

4. A process according to claim 1, characterized in that the ether is diethylene glycol dimethyl ether or dioxan.

5. A process according to Claim 1, characterized in that the molar ratio of lactate (II) to thionyl chloride is from 0.8:1 to 2:1, preferably greater than 1.05:1.

6. A process according to Claim 1, characterized in that the decomposition temperature is from 80°C to the boiling point of the solvent or is less than 140°C if the solvent has a higher boiling point.

7. A process according to any one of Claims 1 to 6, characterized in that the lactate (II) is contacted in a first step with the thionyl chloride to obtain a chlorosulphite of formula:

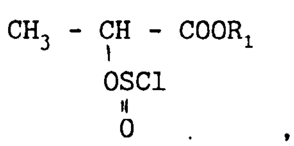

(III)

and the chlorosulphite (III) is then decomposed in the presence of the ether.

8. A process according to Claim 7, characterized in that the chlorosulphite is diluted by the ether to give a solution containing 5 to 95% by weight of chlorosulphite.

9. A process according to Claim 1, characterized in that the lactate (II), the thionyl chloride and the ether are put in contact and then heated.

10. A process according to Claim 9, characterized in that the dilution of the lactate with respect to the ether is 5 to 95% by weight.

**Patentansprüche**

1. Verfahren zur Herstellung von optisch aktiven 2-Chlorpropionsäureestern der Formel

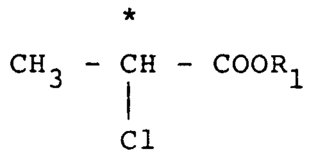

I

wobei $COOR_1$ eine hydrolisierbare Gruppe ist, ausgehend von einem optisch aktiven Lactat der Formel

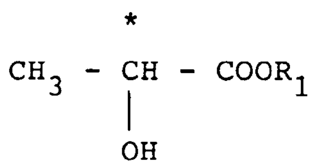

II

unter Inversion der Konfiguration durch In-Kontaktbringen eines Lactats (II) mit $SOCl_2$, gefolgt von einer Zersetzung des gebildeten Chlorsulfits, wobei dieser Prozeß dadurch gekennzeichnet ist, daß wenigstens der Zersetzungsschritt in Gegenwart eines Ethers bewirkt wird.

2. Verfahren nach Anspruch 1, dadurch gekennzeichnet, daß die Gruppe $R_1$ bevorzugt ausgewählt ist aus folgenden Resten:
lineares oder verzweigtes $C_{1-18}$ Alkyl, vorzugsweise $C_{1-12}$ Alkyl,
lineares oder verzweigtes $C_{2-18}$ Alkenyl, vorzugsweise $C_{2-12}$ Alkenyl,
lineares oder verzweigtes $C_{2-18}$ Alkenyl, vorzugsweise $C_{2-12}$ Alkenyl,
lineares oder verzweigtes $C_{3-18}$ Cycloalkyl, vorzugsweise $C_{3-12}$ Cycloalkyl,
$C_{6-14}$ Aryl, vorzugsweise $C_{6-10}$ Aryl,
lineares oder verzweites $C_{7-15}$ Aralkyl, vorzugsweise $C_{7-11}$ Aralkyl,
wobei diese Reste eventuell mit einem oder mehreren Halogenatomen, $C_{1-6}$ Alkoxy- oder Alkyl-thioresten substituiert sind, wobei die aromatischen Ringe der Aryl- oder Aralkylreste als Ersatz von 1 bis 4 Kohlenstoffatomen, weiterhin 1 bis 4 Heteroatomen, ausgewählt unter dem Sauerstoff-, Schwefel- und Stickstoffatom (beispielsweise, Furyl, Thiophenyl, Pyridyl), enthalten können.

3. Verfahren nach Anspruch 2, dadurch gekennzeichnet, daß $R_1$ ein $C_{1-6}$ Alkylrest ist.

4. Verfahren nach Anspruch 1, dadurch gekennzeichnet, daß der Ether aus Diglymen oder Dioxan ausgewählt ist.

5. Verfahren nach Anspruch 1, dadurch gekennzeichnet, daß das Molverhältnis Lactat (II) zu $SOCl_2$ zwischen 0,8 und 2 liegt und vorzugsweise über 1,05 ist.

6. Verfahren nach Anspruch 1, dadurch gekennzeichnet, daß die Zersetzungstemperatur zwischen 80°C und der Siedetemperatur des Lösungsmittels liegt oder unter 140°C, wenn das Lösungsmittel eine höhere Siedetemperatur aufweist.

7. Verfahren nach einem der Ansprüche 1 bis 6, dadurch gekennzeichnet, daß man in einem ersten Schritt das Lactat (II) und Thionylchlorid miteinander in Kontakt bringt, so daß man ein Chlorsulfit der Formel

$$CH_3 - \underset{\underset{OSOCl}{|}}{CH} - COOR_1 \qquad III$$

erhält und dann das Chlorsulfit (III) in Gegenwart eines Ethers zersetzt.

**8.** Verfahren nach Anspruch 7, dadurch gekennzeichnet, daß die Verdünnung des Chlorsulfits bezogen auf den Ether zwischen 5 und 95 Gew.% beträgt.

**9.** Verfahren nach Anspruch 1, dadurch gekennzeichnet, daß man das Lactat (II), den Thionylchlorid und den Ether in Kontakt bringt und dann erhitzt.

**10.** Verfahren nach Anspruch 9, dadurch gekennzeichnet, daß die Verdünnung des Lactats bezogen auf den Ether zwischen 5 und 95 Gew.% beträgt.